# EUROPEAN PATENT SPECIFICATION

(11) **EP 0 379 379 B1**
(45) Date of publication and mention of the grant of the patent: **30.11.1994**
(21) Application number: 90300554.4
(22) Date of filing: 18.01.1990
(51) Int. Cl.: B01J 13/02, A01N 25/00, A01N 25/28, B01J 13/10

(54) **Particulate materials, their production and use**
Granulate, ihre Herstellung und Anwendung
Matières granulaires, leur production et application

(30) Priority: 20.01.1989 GB 8901254
(43) Date of publication of application: 25.07.1990
(73) Proprietor: ALLIED COLLOIDS LIMITED, Bradford, West Yorkshire BD12 0JZ (GB)
(72) Inventor: Chamberlain, Peter, Shipley, West Yorkshire (GB); Langley, John Graham, Shipley, West Yorkshire (GB)
(74) Representative: Lawrence, Peter Robin Broughton

(56) References cited:
- GB-A- 1 275 712
- GB-A- 1 507 739
- GB-A- 2 073 132
- CHEMICAL ABSTRACTS, vol. 80, no. 12, 25th March 1974, page 37, abstract no.60709z, Columbus, Ohio, US; &JP-A-73 55 184 (TOKYO SHIBAURA ELECTRIC CO., LTD) 02-08-1973

## Description

This invention relates to particulate compositions, for instance dispersions, which are convenient and safe to handle and by which active ingredients can be delivered to a chosen place of use. The active ingredients may be, for instance, materials such as pesticides or other agriculturally useful compounds (agrochemicals), pharmaceuticals or perfumes. The invention is of particular value when applied to the formulation of water insoluble pesticides and other water-insoluble, agrochemicals.

It is frequently necessary to formulate the compound as a concentrate that can be diluted at the point of use to form a sprayable composition. When the compound is water soluble, the concentrate can be a concentrated aqueous solution or a water soluble granule or other soluble solid. However many agriculturally useful compounds are insoluble in water. It is generally preferred that they should be applied by spraying an aqueous composition and so the concentrate of water-insoluble active ingredient must be stable and must be capable of easy distribution into water.

One common type is a wettable powder in which powdered insoluble material has been treated to render it wettable, but there is increasing concern about dusting and other environmental problems associated with traditional wettable powders.

Another type is a dispersible paste or cream, usually referred to as a "flowable". This can readily be diluted with water and is reasonably satisfactory for many active ingredients. However a problem with both flowables and wettable powders is that the active ingredient has to be produced in the form of particles of a desired small size and this can be difficult or impossible with some materials, especially some agrochemicals. For instance these formulations are not appropriate when the active ingredient is a liquid or when it is a relatively low melting solid, for instance a solid that melts at below 80°C, because of the difficulty of making the active ingredient in this fine particulate form.

Water insoluble liquids and low melting solids can be formulated as emulsifiable concentrates but it is now frequently considered desirable to avoid this type of formulation for environmental reasons associated with, for instance, the organic solvent that is generally present in such concentrates.

It would therefore be desirable to be able to provide an active ingredient in the form of a particulate composition which is readily dilutable by water, is substantially free of organic solvent or dusting problems, and gives a satisfactory rate of release of the active ingredient.

It is already well known to use polymeric materials in the formulation of various active ingredients. Thus it is known to diffuse a low melting or liquid active ingredient into a preformed polymeric matrix (e.g., as in US-A-4,303,642) or to encapsulate an active ingredient in beads by forming an emulsion or dispersion in water of polymerisable material and the active ingredient and then polymerising the polymerisable material. The product will, depending upon the materials and process conditions, be in the form of either a particulate matrix throughout which the active ingredient is distributed or small (or large) capsules comprising a shell of polymeric material around a core containing the active ingredient.

Although these diffusion and polymerisation techniques are useful in some instances, they are relatively expensive and this is justified only because they are designed primarily to provide controlled release of an active ingredient. They are not appropriate to the more fundamental problem of providing an improved way of formulating a wide range of water insoluble agrochemical or other active ingredients in an economic manner in the form of environmentally satisfactory concentrates that can readily be diluted with water to form sprayable compositions.

There are, of course, numerous other ways of making capsules containing active ingredients.

In GB-A-1,275,712, an active ingredient is dispersed (as an aqueous solution or as a hydrophilic or hydrophobic powder) into a solution of a polymer in an organic solvent, the resultant dispersion is distributed into an aqueous electrolyte and the organic solvent is evaporated to cause deposition of the polymer around the initial particles of powder or aqueous solution. The capsule wall is formed by insolubilisation of the dissolved polymer and surrounds a core consisting of the powder or aqueous solution. The capsules are said to have a size of 20µm to 5mm and are recovered as powder.

Instead of forming a capsule wall by polymerisation or by precipitation of a polymer due to evaporation of its solvent, it is also known to form capsule walls by coacervation. Often at least two water soluble coacervating polymers are blended in aqueous solution, the polymers and their conditions of blending being such that they form a coacervate that comes out of solution to form a coating around a core consisting of one or more particles dispersed in the solution.

In GB-A-1,475,229 a process is described in which microcapsules are formed by dispersing an active ingredient, as a solid or a solution in high boiling (or low boiling) organic solvent, into an aqueous solution of a hydrophilic colloid, enclosing the dispersed particles within a polymeric coating formed by coacervation as a result of the addition of a second coacervating polymer solution, and then cross linking the resultant coating. The resultant microcapsules can have a wide range of particle sizes, namely 0.5 to 500µm. In practice the capsules are separated to form a powder.

Each of GB-A-1,275,712 and 1,475,229 describe the incorporation of a wide range of materials as the active ingredients and included amongst the listed active ingredients are agrochemicals such as pesticides. However in practice techniques such as these do not seem to have been used successfully commercially for the formulation of agriculturally useful active ingredients and instead such methods appear to have been used primarily for encapsulating liquid inks. Numerous other coacervation techniques are described, nearly always with the primary intention of producing and recovering ink capsules that can be applied to paper.

A problem in all coacervation processes is the production of a dispersion of the material that is to be encapsulated (generally the ink) that is sufficiently stable during the coacervation process. Once the coacervation has occurred, it is usually necessary for the encapsulated particles to be further stabilised by wall building using in situ cross linking processes. Methods in which the ink dispersion is stabilised during the coacervation technique by reliance upon polymers that go into the coacervate are described in GB-A-1,507,739, GB-A-2,073,132 and DE-A-3,545,803. Although these processes go through an intermediate stage in which the coacervating polymer contributes to the stability of the ink dispersion, the overall process generally involves conventional cross linking and other wall-building polymerisation processes after the initial coacervation.

Accordingly none of these proposals address or solve the problem of providing a composition of a water insoluble agrochemical or other active ingredient which requires no further polymerisation to stabilise the emulsion and which can readily be diluted, and which is stable during storage.

Another problem with coacervating techniques is that they are of rather limited applicability due to the restrictions imposed by the physical state of the active ingredient that is to be encapsulated. A method is published in WO-A-89 04 714 (after the priority date of this application) for encapsulating low melting materials but there are other materials where this, and other known methods of coacervation, are inappropriate.

In one aspect of the invention, a composition according to the invention is dilutable with water to form a sprayable composition and comprises a substantially stable dispersion in an aqueous medium of particles that are substantially all below 10µm in size and that comprise a core comprising a solid, water-insoluble, polymeric matrix through which is distributed a water insoluble active ingredient surrounded by a shell that has been formed by coacervation from one or more water soluble coacervating polymers and that stabilises the particles against agglomeration in the aqueous medium.

Thus in the invention the coacervating shell is a material that promotes the stability of the particles, as a result of which the coacervated particles have less tendency to settle out and agglomerate than would similarly sized particles of the active ingredient alone, in an otherwise identical aqueous medium. Thus, for the first time, the final product relies upon the coacervate shell to promote the stability of the final product that is to be stored and transported to the point of use, where it can be converted into a novel sprayable composition merely by dilution with water. This is therefore in contrast to processes such as those described in GB-A-1,507,739 where some initial stabilisation is achieved but the process that is conducted involves further reaction of the coacervate shell so as to form a relatively unstable dispersion from which the capsules could be separated relatively easily.

The concentrated composition of the invention must be sufficiently stable that it either remains as a true dispersion during storage or, if settlement does occur, can easily be redispersed merely by stirring. The composition may include additional emulsifiers, dispersants or dispersion stabilisers, but preferably the amount of such materials is very much less, below half and preferably below one quarter, the amount that would be required to stabilise the dispersion in the absence of the stabilising coacervate shell. Preferably the composition is substantially entirely free of such materials although coacervating polymer is preferably dissolved in the solution. Thus the coacervating shell may provide most, and preferably all, of the stabilising properties that are required.

The coacervate shell can be made by bringing the polymers out of solution by, for instance, mixing counterionic polymers that cause mutual insolubilisation either in the presence of dispersed active ingredient or with the subsequent addition of it. Such techniques are known from, for instance, GB-A-2,073,132.

In order that the coacervating shell can provide these properties it is desirable for the shell to be ionically charged, so that the particles then repel one another as a result of the like ionic charges on them. Preferably the particles have a relatively high positive or, preferably, negative zeta potential. Thus the zeta potential is preferably greater than -30mv, and often greater than -50mv, for instance the zeta potential can be as high as -100mv or even higher. This can be achieved by using sufficient of an ionically charged polymer that the ionic charges in the polymer impart the desired zeta potential to the particles, and preferably excess of the ionic polymer is dissolved in the aqueous medium, thus promoting mutual repulsion of the particles. Thus preferably at least two water soluble coacervating polymers are used and one of the coacervating polymers is anionic and the amounts of polymers that are used are such that the particles are rendered anionic and the aqueous medium has some of the anionic polymer in solution in it. The ionically charged polymer should contain a sufficiently high proportion of ionic groups to impart the desired ionic charge to the particles.

The ionic polymer that contributes to the charge of the particles needs to be relatively hydrophilic in order that it gives good dispersing properties but this conflicts with the requirement that it shall come out of solution as a coacervate coating. A preferred way of insolubilising the anionic polymer so as to form a coacervate while still leaving the polymer with hydrophilic properties, is to coacervate it with a non-ionic or, preferably, cationic polymer that has characteristics such that it can interact with the anionic polymer to form the coacervate around the hydrophobic active ingredient but without destroying the dispersing properties of the anionic polymer shell. Preferably the cationic polymer has a lower molecular weight than the anionic polymer and the anionic polymer should be present in a molar excess, that is to say there should be a significantly larger number of anionic groups than cationic groups.

In order that the anionic polymer is hydrophilic, it is preferred to make it from a water soluble ethylenically unsaturated monomer or monomer blend that includes carboxylic or other anionic monomer. Preferably a 20:80 to 80:20 blend of acrylamide and (meth) acrylic acid (usually as sodium salt) is used. The lower molecular weight cationic polymer can be made from water soluble ethylenically unsaturated monomer or monomer blend that includes cationic polymer, a condensation polymer, most preferably a cationised urea formaldehyde or melamine formaldehyde polymer.

Suitable blends of molecular weight can be identified by experimentation, but generally the cationic polymer should have a molecular weight of below 100,000 and in practice the molecular weight should normally be below 50,000 and often below 10,000, while the anionic polymer will normally have a molecular weight above 100,000 and in practice often above 200,000, and up to 1 to 2 million although higher molecular weights can sometimes be used. At least one of the polymers (generally the anionic) can be amphoteric provided this does not prevent the desired formation of a stabilising coacervate.

What seems to be happening is that several molecules of cationic polymer become ionically bonded to parts of an anionic polymer chain so as to render those parts where charge neutralisation occurs more hydrophobic, thus attracting those parts to the hydrophobic active ingredient. The remainder of the anionic polymeric chain remains hydrophilic, thus promoting a high negative zeta potential and good stabilisation properties in an aqueous medium that includes an aqueous solution of the anionic polymer or of some other similar anionic polymer.

Suitable polymeric materials are described in, for instance, DE-A-3,545,803, GB-A-2,073,132, and 1,507,739 and US-A-4,100,103.

The coacervate polymeric coating generally provides at least 10% and often at least 20% by weight of the dry weight of the particles but it is usually unnecessary for it to provide more than 50%, and usually it provides less than 40% of the dry weight of the particles. The content of active ingredient in the aqueous concentrate typically is in the range 5 to 20% by weight based on the concentrate.

In the aqueous dispersions of the invention, at least 90% by weight of the particles should be below about 10µm in size since it will be very difficult to achieve adequate stabilisation if a significant proportion of the particles are above about 10µm, and preferably at least 95% by weight are below 10µm. Preferably at least 90, and usually at least 95%, by weight are below 5µm. At least 50% are preferably below 3µm. The coacervation is preferably conducted so as to make particles that initially have this size, but if necessary the coacervated material can be stirred or milled so as to break agglomerates down to the desired particle size.

The active ingredient can be any material that it is desired to provide in particulate form. Preferably it is an agriculturally useful material such as a semiochemical, nutrient or plant growth regulator or, preferably, a herbicide or pesticide. Suitable pesticides include insecticides, fungicides, nematocides, and biocides. Other suitable active ingredients that can usefully be incorporated into the particulate compositions of the invention include, for instance, perfumes, fragrances, pharmaceuticals and veterinary materials. Preferred active ingredients are chlorpyriphos, chlorpyriphos methyl, and trifluralin.

Although the invention is primarily of value for the production of agrochemical compositions that are stable dispersions that can be diluted with water, it is possible to apply the same coacervation technique to the production of other agrochemical particulate compositions.

In order to perform the coacervation process it is necessary to provide a dispersion of the active ingredient in the desired particulate form, and conventional methods of providing such a dispersion, and conducting the coacervation, tend to be unsatisfactory with many of the agrochemicals and other active ingredients with which we are preferably concerned, even though the methods may be satisfactory for inks. Accordingly we have developed two methods of particular value in the invention.

When the active ingredient is a solid that melts at below 80°C (often below 50°C), the process preferably comprises melting the active ingredient dispersing it and the matrix polymeric material or a precursor of that into the aqueous medium at a temperature at which it remains molten and under conditions to generate particles of the desired size (substantially all below 10µm), coating the resultant dispersed particles by the coacervation process, and cooling the particles. This cooling may occur before the coacervation but after the dispersion stage, but often occurs during or, preferably, after the coacervation. After cooling, the particles are at a temperature below the melting point of the active ingredient.

Another convenient way of performing the process on many agrochemicals and other suitable active ingredients comprises dispersing the active ingredient into the aqueous medium while the active ingredient and the matrix polymeric material or a precursor of that is present as a solution in an organic solvent that is more volatile than water, and thereby forming particles substantially all below 10µm. The dispersed particles are then coated by the coacervation step while dispersed in the aqueous medium, and the resultant dispersion is then distilled to remove the organic solvent.

In this process, the active ingredient can be a liquid at room temperature (20°C) but is generally a solid. It should be soluble in one or more organic solvents. The solution in the organic solvent is preferably a true solution but it can be a partial solution, for instance being a dispersion that is stable in the absence of a dispersion stabiliser. The solubility of the active ingredient in the chosen solvent can be high or low, provided that it is not so low that it is impossible to achieve a solution of useful concentration.

The solvent that is used should be substantially water-immiscible in order that it will emulsify or disperse (rather than dissolve) into the aqueous medium. The dispersion can be achieved by mixing the solution into the aqueous medium under shear using, for instance, a Silverson mixer or other suitable emulsifier or homogeniser apparatus.

The solvent should be significantly more volatile than water and should have a boiling point substantially below the boiling point of water. For instance the solvent preferably has a boiling point below 70°C and preferably between room temperature and 50°C (at atmospheric pressure). Preferably the solvent forms an azeotrope with water so that the removal of solvent by distillation involves azeotropic distillation of substantially all the solvent with part only of the water of the aqueous medium.

Suitable solvents include any of the relatively low boiling water-immiscible organic solvents. The solvent is normally a hydrocarbon or halogenated hydrocarbon, the hydrocarbon generally being aliphatic or cycloaliphatic. Methylene chloride is particularly preferred.

Generally the coacervate coating is substantially complete before the product is distilled, but in some instances it can be desirable to rely upon the heating (that is supplied to cause distillation) also causing or completing the coacervation process.

The evaporation can be conducted by distillation at atmospheric or reduced pressure and is generally conducted as an azeotropic distillation. This is generally conducted under reduced pressure. By appropriate selection of the solvent and the pressure at which distillation is conducted it is possible to effect the distillation at low temperatures, e.g., as low as 50°C or even as low as 30°C, and this is very advantageous if the active ingredient is sensitive to elevated temperatures, for instance being thermally unstable or volatile.

The distillation is preferably continued until the amount of solvent remaining is so low that the resultant composition, even after storage in a closed container, does not have a measurable flash point. Even if some solvent does remain (e.g., 2-30% based on active ingredient and total polymer) the amount is preferably as low as possible so as to maximise the solids content of the composition and to minimise environmental problems due to the solvent.

The distillation conditions must, of course, be such as to remove the solvent whilst allowing sufficient of the aqueous phase to remain. Generally the amount of water in the distilled product is at least 30% and often at least 50%. Solids contents in the range 20% to 60% are suitable.

The coacervate shell can be a discontinuous stabilising shell, for instance a particulate shell of the type formed in DE-A-3,545,803, but preferably the shell is a substantially continuous polymeric coating. We have found that the polymeric coating formed by coacervation does not provide a serious impediment to satisfactory rates of release of the active ingredient to the pest or plant where the desired effect is to be achieved. However in some instances it is desirable to modify the capsules so as to regulate the rate of release.

Whereas it is preferred, from the stabilisation point of view, that the shell of the polymer particles in the dispersion should be the unreacted shell formed by coacervation, it can be desirable to subject the polymer in the shell to further reaction, provided this does not seriously impair the stabilisation properties of the shell. Thus although the shell preferably contains all the unreacted anionic groups initially present at the end of the coacervation process, if desired some of these can be further reacted so as to modify the properties of the shell. For instance they can be subjected to cross-linking with further urea formaldehyde or melamine formaldehyde resin as described in GB-A-1,507,739 or 2,073,132. This will increase the strength of the shell but will reduce the stability of the dispersion and a better way of controlling the rate of release is achieved as a result of the core of the particles comprising the polymeric matrix through which the active ingredient is distributed either as a dispersion or solution in the polymer.

Although the composition can be made by coacervation around, for instance, pre-formed solid particles of the core, it is preferred to form the polymeric coacervate coating around fluid particles. Preferably the active ingredient and any matrix polymeric material (or material that is a precursor of that) are both soluble in an organic solvent that is more volatile than water and the dispersion is made by the solvent process described above. In this type of process, the polymer can be one that is, for instance, a dispersion that is stable in the absence of a dispersion stabiliser in the chosen solvent for the active ingredient or can be a polymeric material that has been formed by reaction in the particles from precursor material that is soluble in the chosen solvent, i.e., the precursor for the matrix polymer must be soluble in the solvent.

When the particles are being made by the melting technique described above then the matrix polymer must be molten at the chosen temperature or must be made from a precursor material that will polymerise during or after dispersing the particles in the aqueous medium.

It is often desirable to make the matrix polymer particles by dispersing precursor polymerisable material and the active ingredient in the aqueous medium and then causing polymerisation or cross linking.

The precursor material can be monomeric or other low molecular weight material but is preferably polymeric. Thus the reaction of the precursor polymer to form the matrix polymer may be, for instance, cross linking or graft polymerisation. When the solvent process is used, the matrix polymer in the final particles is preferably substantially unchanged chemically from the polymer that is dissolved into the initial solution of active ingredient and organic solvent.

The matrix polymer is normally selected such that the core is not only insoluble in the aqueous medium but is also substantially unswollen by the aqueous medium. Suitable polymers are any of the vinyl (or allyl) addition polymers that can conveniently be made by oil-in-water polymerisation or solution polymerisation in an organic solvent of ethylenically unsaturated monomer or monomer blend that is insoluble in water. Preferred polymers are acrylic polymers formed from monomers that comprise alkyl (meth) acrylate, generally in an amount of 30 to 100%. Other monomers that can be included, generally in amounts of less than 70% and preferably less than 40%, include styrenes, acrylonitrile, vinyl halides and the other relatively hydrophobic monomers that conventionally can be included in acrylic oil-in-water emulsion polymerisation. The polymer is generally linear but chain branching or slight cross linking can sometimes be desirable and so cross linking agent may be included.

The preferred matrix polymer is formed from 50 to 100% (preferably 80 to 100%) alkyl (meth) acrylate, and 0 to 50% styrene. Percentages are by weight. The alkyl group generally contains 1 to 10, preferably 2 to 6, carbon atoms. Blends of alkyl (meth) acrylates may be used. Polymers of 80 to 100% isobutyl methacrylate are particularly suitable.

Preferably the matrix polymer has a glass transition point (Tg) of above -20°C and generally above -5°C. It is normally preferred for the glass transition point to be below 100°C and usually below 40°C. By appropriate choice of Tg, monomers, and the ratio by weight of polymer:active ingredient, it is possible to regulate the rate of release.

Preferably the matrix polymer is substantially non-ionic and preferably it is formed entirely from non-ionic material. This is desirable since it facilitates the performance of the manufacturing process and in particular it minimises the risk of undesirable interaction between the matrix polymer and the coacervating polymers. Thus the polymeric matrix should be substantially non-swelling in the aqueous medium, and chemically inert to the coacervating polymers. However it can be convenient for it to swell slightly when the pH is changed, for instance when it is introduced into a more alkaline environment than the aqueous medium in which it is formed. Thus the matrix polymer may include (meth) acrylic or other acid groups and may be in the substantially free acid and non-swollen form in the aqueous medium but may swell slightly when exposed to alkaline conditions, such as may exist in the soil or on a plant leaf, due to ionisation of the carboxylic groups.

Since the matrix polymer (or its precursor) and active ingredient initially are both dissolved in the same solution, they are present in intimate admixture in the final particles and the polymer provides a matrix for the active ingredient.

When polymeric matrix is present, it is generally present in an amount of at least 0.2 parts, preferably at least 0.5 parts, per part dry weight of active ingredient, but it is usually unnecessary for it to provide more than about 2, or at the most about 5, parts by weight of the active ingredient.

The aqueous concentrates of the invention can include conventional additives to improve the stability of the composition (for instance thickeners such as gums or other natural polymers or synthetic polymeric thickeners). It may include dispersants and/or wetters to facilitate its dilution to form a sprayable composition and to facilitate its subsequent spraying and adherence to plants, soil or other substrate to which it is sprayed.

An advantage of the aqueous dispersions of the invention, is that a flowable agrochemical composition, containing particles of a second agrochemical active ingredient, can be blended into the composition. Thus it is possible for the first time to provide in a convenient and environmentally acceptable form a concentrate of two insoluble agrochemicals wherein at least one has physical properties such that it cannot be put into the form of a conventional flowable.

Instead of using the aqueous dispersion as a concentrate for forming a dilute agricultural spray, it can be used for other purposes. For instance the concentrate (or a diluted composition formed from it) may be applied by spraying or otherwise on to a granular or other carrier, for instance to make a dry particulate composition that can be spread as a dry powder. Another way of making a spreadable powder is by aggregating the particles of the dispersion and then drying the aggregates. These aggregates can then be spread as dry powder or can be dispersed into water, whereupon the aggregates will disintegrate.

The following are some examples.

### Example 1

### Preparation of an Aqueous Dispersion of Chlorpyrifos

120g of a 100% polyisobutyl acrylate (as matrix polymer) and 120g Chloropyrifos technical grade were dissolved in 520g dichloromethane, to form Solution A.

168g of a 20% solution of a copolymer of acrylamide/sodium acrylate having molecular weight about 400,000 were dissolved in 600g water (Solution B).

76g of a 35% cationic urea/formaldehyde resin containing about 10 urea units and sold under the trade name BC777 was dissolved in 100g water (Solution C).

Solution B was subjected to stirring by a Silverson stirrer. Solution C was added over 20 secs., stirring was continued for 30 secs., and then solution A was added over 30 secs. Stirring was continued for a further 40 secs., and defoamer was added. A white emulsion was obtained.

The stirred emulsion was subjected to distillation under reduced pressure at a maximum temperature of 45°C, until all the dichlormethane was removed.

After sieving through a nylon mesh, a stable aqueous dispersion of solid particles was obtained and had a solids content of 26.5% by weight. The Chlorpyrifos content of the product was 10.1% and the median particle size was 2.21µm. The zeta potential of the dispersion was -110.4mv.

The dispersion was stable on storage, and was diluted with water to give a 0.3% concentration of particles in the sprayable solution. Some pot-grown cauliflowers were sprayed with the solution while others (the controls) were left untreated. Some of the treated and controlled pots were innoculated with cabbage root fly eggs immediately while others were innoculated after 5, 10, 15 or 22 weeks, and the pots were then grown in an insectary whilst the cabbage root fly larvae pupated. The mean numbers of larvae and pupae in each pot were counted. The results were as follows.

| Week | 0 | 5 | 10 | 15 | 22 |
|---|---|---|---|---|---|
| Control-number | 5.0 | 9.6 | 5.4 | 5.7 | 0.3 |
| Sample-number | 0 | 0 | 0.1 | 0 | 0 |

This demonstrates that the active ingredient was effective both immediately and for a period of 22 weeks, the maximum useful life of the crop.

### Examples 2, 3 and 4

The process was repeated in a manner similar to that, with varying amounts of matrix polymer: 0g, 60g, 240g were added. In each case a stable dispersion was obtained.

### Example 5

### Preparation of an Aqueous Dispersion of 1,7-Dioxaspiro (5,5) Undecane ('Spiroketal')

Spiroketal is the principle component of the sex pheromone of the Olive fly : Dacus oleae.

160g of a 95:5 copolymer of methyl methacrylate:ethyl acrylate and 22.5g Spiroketal were dissolved in 640g dichlormethane to form Solution A.

Solution B and C were prepared as in Example 1.

The solutions A, B and C were mixed in a manner identical to Example 1.

the dichloromethane was removed by distillation under reduced pressure in the temperature range 40-50°C.

The final product was a stable dispersion of white particles of <5µm size.

### Example 6

This was identical to Example 5, except that the polyisobutyl acrylate of Example 1 was used in place of the 95:5 copolymer.

It was found that the rate of release of the spiroketal to the atmospher was different in the two products.

### Example 7

Solutions B and C were made and mixed as in Example 1, and the mixed solution was then heated to 45°C and 800g molten Trifluralin (technical grade) was added at 45°C and blended using a Silversen stirrer. The liquid was allowed to cool with gentle sitrring and was then subjected to stirring with a Silversen stirrer until 90% of the particles were below 10µm and 50% were below 5µm. The product was a stable orange suspension containing 45% by weight Fluralin.

### Example 8

A conventional flowable containing 50% by weight Linuron was made by bead milling a mixture of 538g Linuron (technical grade) 253g water, 138g monoethylene glycol, 69g surfactant and 1g antifoam, until the particle size of the flowable was 100% below 10µm. 150g of this flowable suspension was then mixed with 333g of the Trifluralin suspension of Example 7 and 76g water to give a stable suspension containing 300g/l Trifluralin and 150g/l Linuron.

## Claims

1. A composition that is dilutable with water to form a sprayable composition and that comprises a substantially stable dispersion in an aqueous medium of particles that are substantially all below 10µm in size and that comprise a core comprising a solid, water-insoluble, polymeric matrix through which is distributed a water insoluble active ingredient surrounded by a shell that has been formed by coacervation from one or more water soluble coacervating polymers and that stabilises the particles against agglomeration in the aqueous medium.

2. A composition according to claim 1 in which the active ingredient is selected from solids that have a melting point below 80°C and liquids.

3. A composition according to claim 1 or claim 2 in which the active ingredient is selected from agrochemicals, pharmaceuticals, veterinary chemicals, perfumes and fragrances.

4. A composition according to any preceding claim in which the active ingredient is an agrochemical selected from herbicides, semiochemicals, plant growth regulators, nutrients, and pesticides.

5. A composition according to claim 1 in which the active ingredient is selected from chlorpyriphos, chlorpyriphos methyl and trifluralin.

6. A composition according to any preceding claim in which one of the coacervating polymers is anionic and renders the particles anionic, and the said anionic polymer is also dissolved in the aqueous medium.

7. A composition according to any preceding claim in which the shell is formed by coacervation of a low molecular weight cationic polymer with a molar excess of a higher molecular weight water soluble anionic polymer.

8. A composition according to any preceding claim in which the shell is the unreacted shell that has been formed by coacervation of a low molecular weight, cationic polymer and a molar excess of a hydrophilic, higher molecular weight, anionic polymer.

9. A composition according to any preceding claim in which the shell is formed by coacervation of cationic urea formaldehyde or cationic melamine formaldehyde having a molecular weight of below 100,000 with a molar excess of water soluble anionic polymer of ethylenically unsaturated carboxylic acid and having a molecular weight above 100,000.

10. A composition according to any of claims 6 to 9 in which the anionic polymer is a copolymer of acrylamide and acrylic acid.

11. A composition according to any preceding claim in which the active ingredient is an agrochemical and a flowable, containing particles of a second agrochemical active ingredient, has been blended into the composition.

12. A composition according to any preceding claim in which the dispersion of the particles containing the core and shell has been made by dispersing into an aqueous medium a solution of water insoluble active ingredient dissolved in an organic solvent that is more volatile than water, and thereby forming particles substantially all below 10µm, coating the resultant dispersed particles by coacervation of the coacervating polymers and thereby stabilising the particles against agglomeration while dispersed in the aqueous medium, and distilling the dispersion to remove the organic solvent.

13. A composition according to any of claims 1 to 11 in which the active ingredient is a solid that melts at below 80°C and the dispersion of the particles containing the core and shell has been made by melting the active ingredient, dispersing it into an aqueous medium at a temperature at which it remains molten and under conditions to generate particles that are substantially all below 10µm in size, coating the resultant dispersed particles by coacervation of at least two water soluble coacervating polymers and thereby stabilising the particles against agglomeration while dispersed in the aqueous medium, and cooling the particles before, during or after the coacervation to a temperature below the melting point of the active ingredient.

14. A composition according to claim 1 that comprises particles that comprise a core comprising a water insoluble agrochemical surrounded by a shell that has been formed by coacervation of a low molecular weight water soluble cationic polymer with a molar excess of a higher molecular weight water soluble anionic polymer.

15. A composition according to claim 14 in which the shell has been formed by coacervation of cationic urea formaldehyde or cationic melamine formaldehyde having a molecular weight of below 100,000 with a molar excess of water soluble anionic polymer of ethylenically unsaturated carboxylic acid and having molecular weight above 100,000.

16. A particulate composition according to claim 1 comprising particles that comprise a core containing a water insoluble active ingredient surrounded by a coacervate shell and which has been made by dispersing into an aqueous medium a solution of water insoluble active ingredient dissolved in an organic solvent that is more volatile than water, coating the resultant dispersed particles by coacervation of one or more coacervating polymers dissolved in the water, and distilling the dispersion to remove the organic solvent.

## Patentansprüche

1. Zusammensetzung, die mit Wasser unter Bildung einer sprühbaren Zusammensetzung verdünnbar ist und welche umfaßt eine im wesentlichen stabile Dispersion in einem wäßrigen Medium von Teilchen, die im wesentlichen alle kleiner als 10 µm sind und welche umfassen einen Kern umfassend eine feste wasserunlösliche polymere Matrix, durch welche ein wasserunlöslicher Wirkstoff verteilt ist, umgeben von einer Hülle, die gebildet wurde durch Koazervation aus einem oder mehreren wasserlöslichen koazervierenden Polymeren und welche die Teilchen gegen Agglomeration in dem wäßrigen Medium stabilisiert.

2. Zusammensetzung nach Anspruch 1, worin der Wirkstoff ausgewählt ist aus Feststoffen mit einem Schmelzpunkt unter 80°C und Flüssigkeiten.

3. Zusammensetzung nach Anspruch 1 oder Anspruch 2, worin der Wirkstoff ausgewählt ist aus Agrochemikalien, Arzneimitteln, Veterinärchemikalien, Parfums und Duftstoffen.

4. Zusammensetzung nach irgendeinem vorangehenden Anspruch, worin der Wirkstoff eine Agrochemikalie ist, ausgewählt aus Herbiziden, Geruchsstoffen, Pflanzenwuchsregulatoren, Nährstoffen und Pestiziden.

5. Zusammensetzung nach Anspruch 1, worin der Wirkstoff ausgewählt ist aus Chlorpyriphos, Chlorpyriphos-Methyl und Trifluralin.

6. Zusammensetzung nach irgendeinem vorangehenden Anspruch, worin eines der koazervierenden Polymeren anionisch ist und die Teilchen anionisch macht, und das anionische Polymer ebenfalls in dem wäßrigen Medium gelöst wird.

7. Zusammensetzung nach irgendeinem vorangehenden Anspruch, worin die Hülle durch Koazervation eines niedermolekularen kationischen Polymers mit einem molaren Überschuß eines höhermolekularen wasserlöslichen anionischen Polymers gebildet wird.

8. Zusammensetzung nach irgendeinem vorangehenden Anspruch, worin die Hülle die nicht-reagierte Hülle ist, welche gebildet wurde durch Koazervation eines niedermolekularen kationischen Polymers und eines molaren Überschusses eines hydrophilen höhermolekularen anionischen Polymers.

9. Zusammensetzung nach irgendeinem vorangehenden Anspruch, worin die Hülle gebildet wird durch Koazervation von kationischem Harnstoff-Formaldehyd oder kationischem Melamin-Formaldehyd mit einem Molekulargewicht unter 100000 mit einem molaren Überschuß eines wasserlöslichen anionischen Polymers einer ethylenisch ungesättigten Carbonsäure mit einem Molekulargewicht über 100000.

10. Zusammensetzung nach irgendeinem der Ansprüche 6 bis 9, worin das anionische Polymer ein Copolymer von Acrylamid und Acrylsäure ist.

11. Zusammensetzung nach irgendeinem vorangehenden Anspruch, worin der Wirkstoff eine Agrochemikalie ist und ein fließbarer Wirkstoff, der Teilchen einer zweiten Agrochemikalie enthält, der Zusammensetzung zugemischt worden ist.

12. Zusammensetzung nach irgendeinem vorangehenden Anspruch, worin die Dispersion der Teilchen, welche den Kern und die Hülle enthalten, erhalten worden ist durch Dispergieren einer Lösung des wasserunlöslichen Wirkstoffs, gelöst in einem organischen Lösungsmittel, das flüchtiger als Wasser ist, in einem wäßrigen Medium unter Bildung von Teilchen, die im wesentlichen alle kleiner als 10 µm sind, Beschichten der erhaltenen dispergierten Teilchen durch Koazervation der koazervierenden Polymeren und dadurch Stabilisieren der Teilchen gegen Agglomeration, während sie in dem wäßrigen Medium dispergiert sind, und Destillieren der Dispersion, um das organische Lösungsmittel zu entfernen.

13. Zusammensetzung nach irgendeinem der Ansprüche 1 bis 11, worin der Wirkstoff ein Feststoff ist, der unter 80°C schmilzt, und die Dispersion der Teilchen, welche den Kern und die Hülle enthalten, erhalten worden ist durch Schmelzen des Wirkstoffs und Dispergieren in ein wäßriges Medium bei einer Temperatur, bei welcher er geschmolzen bleibt und unter Bedingungen, bei denen Teilchen erzeugt werden, die im wesentlichen alle kleiner als 10 µm sind, Beschichten der erhaltenen dispergierten Teilchen durch Koazervation von mindestens zwei wasserlöslichen koazervierenden Polymeren und dadurch Stabilisieren der Teilchen gegen Agglomeration, während sie in dem wäßrigen Medium dispergiert sind, und Abkühlen der Teilchen vor, während oder nach der Koazervation auf eine Temperatur unter dem Schmelzpunkt des Wirkstoffs.

14. Zusammensetzung nach Anspruch 1, welche Teilchen umfaßt, die einen Kern umfassen, umfassend eine wasserlösliche Agrochemikalie, umgeben von einer Hülle, die gebildet worden ist durch Koazervation eines niedermolekularen wasserlöslichen kationischen Polymers mit einem molaren Überschuß eines höhermolekularen wasserlöslichen anionischen Polymers.

15. Zusammensetzung nach Anspruch 14, worin die Hülle gebildet worden ist durch Koazervation von kationischem Harnstoff-Formaldehyd oder kationischem Melamin-Formaldehyd mit einem Molekulargewicht unter 100000 mit einem molaren Überschuß eines wasserlöslichen anionischen Polymers einer ethylenisch ungesättigten Carbonsäure mit einem Molekulargewicht über 100000.

16. Teilchenförmige Zusammensetzung nach Anspruch 1, umfassend Teilchen, welche einen Kern umfassen, der einen wasserunlöslichen Wirkstoff enthält, umgeben von einer Koazervathülle, und welche erhalten worden ist durch Dispergieren einer Lösung des wasserunlöslichen Wirkstoffs, gelöst in einem organischen Lösungsmittel, das flüchtiger als Wasser ist, in einem wäßrigen Medium, Beschichten der erhaltenen dispergierten Teilchen durch Koazervation von einem oder mehreren koazervierenden Polymeren, gelöst in dem Wasser, und Destillieren der Dispersion, um das organische Lösungsmittel zu entfernen.

## Revendications

1. Composition délayable dans l'eau pour former une composition pulvérisable et comprenant une dispersion pratiquement stable dans un milieu aqueux de particules qui ont pratiquement toutes une dimension inférieure à 10 µm et qui comprennent un coeur constitué d'une matrice polymère solide insoluble dans l'eau, dans laquelle est distribué un composant actif insoluble dans l'eau, entouré par une enveloppe qui a été formée par coacervation d'un ou plusieurs polymères de coacervation solubles dans l'eau et qui stabilise les particules contre une agglomération dans le milieu aqueux.

2. Composition selon la revendication 1, dans laquelle le composant actif est choisi parmi des matières solides qui possèdent un point de fusion inférieur à 80°C et des liquides.

3. Composition selon la revendication 1 ou la revendication 2, dans laquelle le composant actif est choisi parmi des produits agrochimiques, des produits pharmaceutiques, des produits chimiques vétérinaires, des parfums et des fragrances.

4. Composition selon l'une quelconque des revendications précédentes, dans laquelle le composant actif est un produit agrochimique choisi parmi des herbicides, des produits semiochimiques, des régulateurs de croissance de végétaux, des substances nutritives et des pesticides.

5. Composition selon la revendication 1, dans laquelle le composant actif est choisi parmi les suivants : chlorpyrifos, chlorpyrifos-méthyle et trifluraline.

6. Composition selon l'une quelconque des revendications précédentes, dans laquelle un des polymères de coacervation est anionique et rend les particules anioniques et dans laquelle on dissout également ledit polymère anionique dans le milieu aqueux.

7. Composition selon l'une quelconque des revendications précédentes, dans laquelle on forme l'enveloppe par coacervation d'un polymère cationique de masse moléculaire faible en présence d'un excès molaire d'un polymère anionique soluble dans l'eau de masse moléculaire plus élevée.

8. Composition selon l'une quelconque des revendications précédentes, dans laquelle l'enveloppe consiste en l'enveloppe n'ayant pas réagi, qui a été formée par coacervation du polymère cationique de masse moléculaire faible et d'un excès molaire du polymère anionique hydrophile de masse moléculaire plus élevée.

9. Composition selon l'une quelconque des revendications précédentes, dans laquelle on forme l'enveloppe par coacervation d'urée-formaldéhyde cationique ou de mélamine-formaldéhyde cationique possédant une masse moléculaire inférieure à 100 000 en présence d'un excès molaire d'un polymère anionique soluble dans l'eau d'un acide carboxylique éthyléniquement insaturé et possédant une masse moléculaire supérieure à 100 000.

10. Composition selon l'une quelconque des revendications 6 à 9, dans laquelle le polymère anionique est un copolymère d'acrylamide et d'acide acrylique.

11. Composition selon l'une quelconque des revendications précédentes, dans laquelle le composant actif est un produit agrochimique et dans laquelle on a mélangé dans la composition un produit fluide contenant des particules d'un deuxième composant actif agrochimique.

12. Composition selon l'une quelconque des revendications précédentes, dans laquelle on a produit la dispersion des particules contenant le coeur et l'enveloppe par dispersion dans un milieu aqueux d'une solution du composant actif insoluble dans l'eau dissous dans un solvant organique qui est plus volatil que l'eau, formant ainsi des particules ayant pratiquement toutes une dimension inférieure à 10 µm, par revêtement des particules dispersées résultantes par coacervation des polymères de coacervation, stabilisant ainsi les particules contre une aglomération alors qu'elles sont dispersées dans le milieu aqueux et par distillation de la dispersion pour éliminer le solvant organique.

13. Composition selon l'une quelconque des revendications 1 à 11, dans laquelle le composant actif est un solide qui fond à une température inférieure à 80°C et dans laquelle on a produit la dispersion des particules contenant le coeur et l'enveloppe par fusion du composant actif, par sa dispersion dans un milieu aqueux à une température à laquelle il reste fondu et dans des conditions permettant de former des particules qui ont pratiquement toutes une dimension inférieure à 10 µm, par revêtement des particules dispersées résultantes par coacervation d'au moins deux polymères de coacervation solubles dans l'eau, stabilisant ainsi les particules contre une aglomération alors qu'elles sont dispersées dans le milieu aqueux et par refroidissement des particules avant, pendant ou après la coacervation à une température inférieure au point de fusion du composant actif.

14. Composition selon la revendication 1, comprenant des particules qui comprennent un coeur contenant un produit agrochimique insoluble dans l'eau, entouré par une enveloppe qui a été formée par coacervation d'un polymère cationique soluble dans l'eau de masse moléculaire faible en présence d'un excès molaire d'un polymère anionique soluble dans l'eau de masse moléculaire plus élevée.

15. Composition selon la revendication 14, dans laquelle on a formé l'enveloppe par coacervation d'urée-formaldéhyde cationique ou de mélamine-formaldéhyde cationique possédant une masse moléculaire inférieure à 100 000 en présence d'un excès molaire d'un polymère anionique soluble dans l'eau d'un acide carboxylique éthyléniquement insaturé et possédant une masse moléculaire supérieure à 100 000.

16. Composition particulaire selon la revendication 1, comprenant des particules qui comprennent un coeur contenant un composé actif insoluble dans l'eau, entouré par une enveloppe de coacervat et qu'on a préparé par dispersion dans un milieu aqueux d'une solution du composé actif insoluble dans l'eau, dissous dans un solvant organique qui est plus volatil que l'eau, par revêtement des particules dispersées résultantes par coacervation d'un ou plusieurs polymères de coacervation dissous dans l'eau et par distillation de la dispersion pour éliminer le solvant organique.
